(19) Europäisches Patentamt
European Patent Office
Office européen des brevets

(11) Numéro de publication: **0 139 554**
**B1**

(12) # FASCICULE DE BREVET EUROPÉEN

(45) Date de publication du fascicule du brevet: **02.08.89**

(51) Int. Cl.⁴: **C 07 K 9/00,** A 61 K 37/02 // C07J41/00

(21) Numéro de dépôt: **84401681.6**

(22) Date de dépôt: **16.08.84**

(54) **Dérivés de Muramyl-peptides et de stéroides ayant des propriétés d'activation des macrophages.**

(30) Priorité: **16.08.83 FR 8313333**

(43) Date de publication de la demande: **02.05.85 Bulletin 85/18**

(45) Mention de la délivrance du brevet: **02.08.89 Bulletin 89/31**

(84) Etats contractants désignés: **AT BE CH DE FR GB IT LI LU NL SE**

(56) Documents cité:
**EP-A-0 004 512**

**JOURNAL OF MEDICINAL CHEMISTRY, vol. 23, no. 11, novembre 1980, pages 1184-1188, Washington, D.C., US; M.M. PONPIPOM et al.: "Glycolipids as potential immunologic adjuvants"**
**EXPERIENTIA, vol. 34, no. 10, 15 octobre 1978, pages 1363-1364, Birkhäuser Verlag, Bâle, CH; K. MASEK et al.: "Immunoadjuvant activity of synthetic N-acetyl muramyl dipeptide"**

(73) Titulaire: **ANVAR Agence Nationale de Valorisation de la Recherche, 43, rue Caumartin, F-75436 Paris Cédex 09 (FR)**

(72) Inventeur: **Tenu, Jean- Pierre, 3, Square des Colonnes, F-92360 Meudon La Foret (FR)**
Inventeur: **Bernard, Jean- Marie, 7, Square Couperin Résidence du Parc, F-78330 Fontenay Le Fleury (FR)**
Inventeur: **Petit, Jean- François, 24, rue Ernest Renan, F-75015 Paris (FR)**
Inventeur: **Phillips, Nigel, 29, Square des Marronniers, F-78870 Bailly (FR)**

(74) Mandataire: **Gutmann, Ernest, S.C. Ernest Gutmann - Yves Plasseraud 67, boulevard Haussmann, F-75008 Paris (FR)**

**EP 0 139 554 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

L'invention concerne des dérivés de muramyl-peptides et de stéroïdes, plus particulièrement de stérols ayant des propriétés d'activateurs de macrophages. Elle concerne plus particulièrement des compositions contenant des dérivés de muramyl-peptides et de stéroïdes, de préférence de stérols, exerçant une stimulation des mécanismes de résistance antitumorale non spécifique in vivo.

L'activation des macrophages est l'un des principaux mécanismes de l'activité antitumorale des immunomodulateurs: les macrophages activés sont capables de détruire des cellules tumorales syngéniques non seulement in vitro, mais aussi in vivo (1). On se reportera à la bibliographie ajoutée à la fin de cette description pour ce qui est des documents de l'état de la technique auxquels il est fait référence par l'intermédiaire de chiffres placés entre des parenthèses.

Il a eté montré (2, 3) que les muramylpeptides pouvaient augmenter l'activité antitumorale des macrophages in vitro. Il n'en est pas de même in vivo lorsque ces substances sont utilisées en solution saline (4): cette inactivité est sans doute due au fait que les muramylpeptides pénètrent lentement dans les macrophages, sans doute par pinocytose fluide (5), et que les muramylpeptides sont éliminés rapidement de l'organisme par le rein (6, 7). Il résulterait alors de la conjonction de ces deux phénomènes que les muramylpeptides injectés en solution saline n'atteignent pas une concentration suffisante dans les macrophages pour pouvoir les activer.

Plusieurs solutions peuvent être envisagées pour remédier à ces inconvénients, en particulier l'encapsulation des muramylpeptides dans des liposomes qui seront phagocytes et délivreront leur contenu à l'intérieur même de la cellule.

FIDLER et ses collaborateurs ont dévelopé cette approche par l'utilisation de liposomes multilamellaires composés de phosphatidylcholine (PC) et de phosphatidylsérine (PS) dans un rapport 7/3 et incluant du MDP; ils arrivent ainsi à cibler cet immunomodulateur vers les monocytes circulants qui se différencient, sous l'influence du MOP qu'ils ont endocyté, en macrophages activés (8, 9, 10).

La composition des liposomes et leur nature (multilamellaires) permettent de les diriger, en particulier, vers les capillaires de la circulation pulmonaire (8, 9, 10). Les monocytes qui les ont phagocytés migrent ensuite dans le poumon où ils se différencient en macrophages activés: ces macrophages activés sont capables de détruire des métastases de tumeurs à tropisme pulmonaire comme le mélanome $B_{16}$ chez la souris (1, 4, 10).

Mais l'utilisation de muramylpeptides solubles se heurte à de nombreux inconvénients: certains liposomes (en particulier ceux dont la composition favorise le ciblage vers les monocytes de la circulation pulmonaire fuient, c'est-à-dire qu'ils perdent le soluté encapsulé: cette fuite est particulièrement marquée lorsque les liposomes PC/PS sont mis en présence de sérum.

Ces fuites interdisent d'autre part une bonne conservation des liposomes,

Cet inconvénient est partiellement surmonté par l'utilisation de liposomes multilamellaires. On peut en effet penser que les espaces interlamellaires les plus internes peuvent contenir ou limiter les fuites avant la phagocytose du liposome. Cette solution entraîne cependant une perte de spécificité du ciblage. En effet l'utilisation de liposomes unilamellaires ou constitués de peu de lamelles pourrait présenter des avantages pour le ciblage vers d'autres organes que le poumon (8).

Pour y remédier FIDLER et ses collaborateurs ont également déjà préconisé l'utilisation de dérivés lipophiles de la N-acétyl-muramyl-L-alanyl-D-isoglutamine (MDP) ou de la N-acetyl-muramyl-L-alanyl-D-isoglutamyl-L-alanine (MTP), tels que le MTP-phosphatidyl-éthanolamine.

L'invention a pour but de remédier encore plus efficacement aux difficultés qui ont éte évoquées ci-dessus, en particulier de fournir de nouveaux dérivés de MDP, possédant une capacité importante d'activation des macrophages, et plus particulièrement de leur activité tumoricide in vivo, et ce plus particulièrement lorsqu'ils sont administrés sous forme de liposomes.

Les dérivés du MDP selon l'invention résultent essentiellement d'une conjugaison covalente d'un muramylpeptide comportant une fraction amine et d'un stéroïde à tropisme membranaire comportant une fonction hydroxyle ou amine, tel que le cholestérol ou un précurseur de sa biosynthèse ou encore un sitostérol, un stigmastérol, la prégnenolone, l'androstérone et l'oestrone.

Des composés préférés de l'invention sont caractérisés par la formule générale suivante

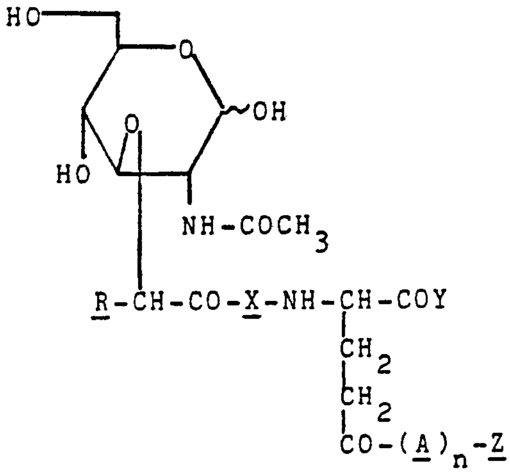

dans laquelle les substituants R, X, Y, A et Z ont l'une des significations suivantes:

- R est soit un atome d'hydrogène, soit un groupement alcoyle comprenant de 1 à 5 atomes de carbone;
- X est un résidu aminoacyle du groupe comprenant: L-alanyl, glycyl, L-valyl, L-isoleucyl, L-norleucyl, L-leucyl, L-seryl, L-threonyl, L-prolyl, L-glutaminyl, L-asparaginyl, L-methionyl, L-tryptophanyl, L-phenylalanyl, L-tyrosyl;
- Y est un groupement $NH_2$ ou OH, ou encore un résidu alkyle comprenant de 1 à 10 atomes de carbone:
- n = 0 ou un nombre entier de 1 à 5:
- A est (lorsque n prend les valeurs de 1 à 5) un résidu aminoacyle du groupe indiqué ci-dessus, mais aussi de formule telle que $-NH-(CH_2)_x-CO-$, avec des valeurs de x comprises entre 2 et 10, étant entendu que les groupes A présents dans un même composé peuvent être identiques ou différents;
- Z est un dérivé de 3-hydroxy-androstane ou de 3-hydroxy-androstène portant en $C_{17}$ une fonction cétone ou une chaîne hydrocarbonée comprenant de 1 à 10, notamment de 2 à 6 atomes de carbone.

Avantageusement, ladite chaîne hydrocarbonée est une chaîne aliphatique, le cas échéant modifiée ou substituée par une ou plusieurs fonctions cétone, ol, amine.

La liaison covalente entre le groupe muramylpeptide et le dérivé de 3-hydroxy-androstène ou de 3-hydroxy-androstène se fait de préférence au niveau de ce groupe hydroxy.

On peut utiliser de préférence le cholestérol, mais aussi d'autres stérols ou stéroïdes pour former les composés selon l'invention tels que les sitostérols, le stigmastérol, la prégnenolone. Il sera avantageux, chaque fois que l'on voudra cibler vers un organe particulier, de mettre en oeuvre dans la constitution du conjugué stérol-muramyl-peptide, celles des hormones stéroïdiques qui sont reconnues, notamment par des récepteurs cellulaires de reconnaissance spécifiques à ces organes. On mentionnera par exemple l'androstérone et l'oestrone.

Des composés particulièrement préférés de l'invention sont constitués par des dérivés muramyl-dipeptide-(λ)-L-alanyl-cholesteryl esters et tout particulièrement le N-acétyl-muramyl-L-alanyl-D-isoglutaminyl-L-alaninecholestéryl ester, de formule

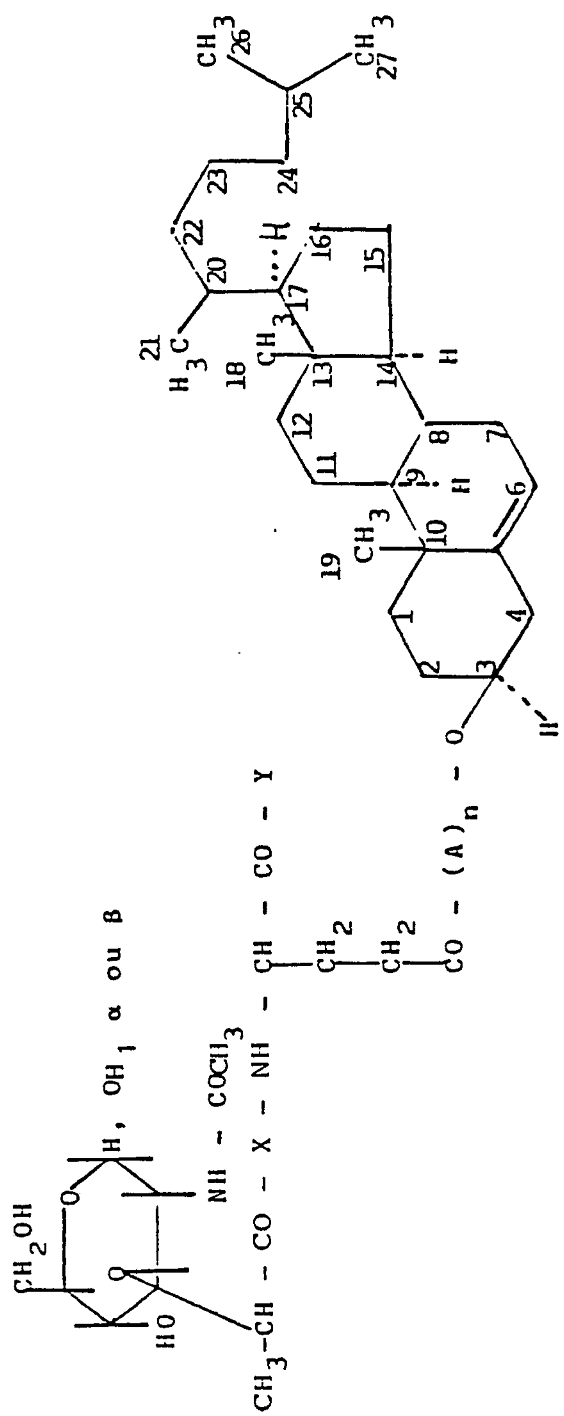

dans laquelle n, A et Y ont les significations sus-indiquées.

De préférence les composés selon l'invention sont utilisés dans des liposomes ou des suspensions de ces liposomes dans des solutions aqueuses physiologiquement acceptables, de préférence stériles et isotoniques, lorsque ces compositions sont destinées à être administrées par voie parentérale.

En ce qui concerne la composition lipidique mise en jeu pour former les liposomes, on peut se reporter à la littérature, notamment celle dont les références bibliographiques sont présentées à la fin de cette description. Des compositions lipidiques préférées sont celles qui mettent en jeu des phospholipides, plus particulièrement des mélanges de phosphatidylcholine (PC) et de phosphatidylsérine (PS). Avantageusement les liposomes sont formés à partir de mélanges contenant les derniers phospholipides mentionnés dans un rapport de 7 volumes de PC à 3 volumes de PS ou d'autres proportions de lipides telles qu'elles sont utilisées pour faire des liposomes selon la cible envisagé. Les activités biologiques liposomes contenant les dérivés de l'invention se manifestent aussi bien lorsque les liposomes se présentent sous forme unilamellaire ou plurilamellaire.

Les produits selon l'invention sont préparés par synthèse.

Pour aboutir à un même composé, diverses voies sont possibles. Dans tous les cas, la synthèse comporte une série d'étapes au cours desquelles les différents "fragments" constituant la structure d'ensemble des composés selon l'invention sont progressivement assemblés. Les principales différences entre les voies possibles se situent dans la séquence choisie pour l'assemblage des fragments. Les modes de réaction conduisant à la fixation d'un fragment au(x) fragment(s) contigu(s) sont dans l'ensemble peu modifiés par l'ordre dans lequel cette intégration est conduite, à ceci près bien entendu que de cet ordre dépend, d'une part, le choix des groupes fonctionnels qui réagissent et qui, par conséquent, doivent être libres pour l'étape considérée, et, d'autre part, le choix des groupes qui doivent être bloqués pour ne pas intervenir au cours de cette même étape.

La préparation des produits selon l'invention peut se faire à partir des composés correspondants du type muramylpeptide. L'obtention de ces derniers a été décrite dans de nombreuses publications, plus particulièrement en ce qui concerne les préparations de l'acide muramique, de ses analogues, ou de ses dérivés, lesquels ont en commun la structure

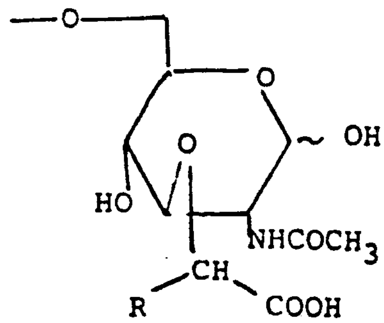

dans laquelle R a la signification précédemment indiquée.

Avantageusement les composés selon l'invention peuvent être synthétisés selon de nombreuses méthodes. On indique ci-après quelques procédés préférés susceptibles d'être utilisés.

La fixation d'une chaîne peptidique sur un dérivé d'acide N-acétyl-muramique, ou d'un analogue de celui-ci tels qu'ils ont été indiqués ce-dessus, est obtenue par des méthodes traditionnelles dans le domaine de la synthèse des peptides. De telles méthodes ont été amplement décrites dans la littérature antérieure et en particulier dans les demandes de brevets français dont les références sont rappelées plus loin.

De façon générale, les synthèses glycopeptidiques peuvent être faites soit en fixant un premier aminoacide au groupe muramyle, puis en fixant au composé ainsi obtenu le second aminoacide, et ainsi de suite de proche en proche. Il est aussi possible de préparer séparément la chaîne peptidique entière aminoacide par aminoacide, puis de fixer celle-ci sur le groupe muramyle. Il est aussi possible de choisir des procédés intermédiaires dans lesquels on prépare des fragments de la chaîne, puis soit d'assembler ces fragments entre eux jusqu'à former la chaîne complète qui est ensuite fixée au groupe muramyle, soit de fixer un premier fragment au groupe muramyle, puis un second au produit ainsi obtenu, etc... Le choix de la séquence est guidé principalement par des raisons de commodité ou de rendement.

La substitution <u>Y</u> est avantageusement réalisée sur le groupe glutamyle avant la synthèse de la chaîne. De la même façon que <u>n</u> soit égal ou différent de 0, le groupe <u>Z</u> est de préférence d'abord fixé à l'amin oacyle terminal avant que celui-ci ne soit intégré dans la chaîne peptidique.

Les synthèses peptidiques sont réalisées suivant des méthodes traditionnelles. A titre d'exemple, on peut choisir les méthodes d'activation des carboxyles, comme la méthode des esters activés ou celle dite des anhydrides mixtes, ou bien celle utilisant un composé du type carbodiimide tel que le N,N'-dicyclohexylcarbodiimide ou des carboiimides équivalents. On trouvera une revue des modes de synthèse peptidique traditionnels dans J.H. JONES, Chemistry and Industry, 723 (1974). On peut aussi se reporter aux demandes de brevets français déjà citées, ou encore aux demandes suivantes: 7 529 624, 7 606 819, 7 606 820, 7 606 821, 7 621

889, 7 702 646, et aux articles de LEFRANCIER et al. (Int. J. Peptide Protein Res., 1977, 9, 249 et 1978, 11, 289).

La formation des dérivés estérifiés ou amidés correspondant au groupe Y est obtenue de façon connue. On peut en particulier se reporter aux demandes de brevets français indiquées ci-dessus, et notamment aux demandes 7 606 820, 7 606 821, 7 621 889 et 7 702 646.

Pour fixer le reste Z via sa fonction hydroxyle à l'acide aminé situé à l'extrémité C-terminale de la chaîne peptidique, on procède à une activation de la fonction carboxylique de cet acide aminé, selon des méthodes bien connues en Synthèse peptidique pour conduire à la formation d'une liaison ester (voir ci-dessous).

a) Séquence de synthèse des composés glycopeptidiques correspondant à la variante de la formule générale dans laquelle n est différent de zéro

L'acide aminé C-terminal est substitué sur sa fonction amine par un groupement de protection temporaire, tel que le t-butyloxycarbonyl (BOC), ou tout autre groupement communémemt utilisé à cet effet en synthèse peptidique. Sa fonction carboxylique est alors activée de façon à se conjuguer avec la fonction hydroxyle du résidu Z, formant ainsi une fonction ester. Diverses méthodes peuvent être utilisées: celles au chlorure de benzène sulfonyle (M. SHEMYAKIN, Angew. Chem. 72 (1960) 342), aux anhydrides mixtes (M. BRENNER, J.P. ZIMERMANN, P. QUITT, W. SCHNEIDER, and A. HARTMANN, Helv. Chim. Acta. 40 (1951 604), au carbonyldiimidazole (H.A. STAAB, ROHR W, MANNSCHRECK A. Angew. Chem. 73 (1961) 143), aux esters activés en présence de catalyseurs, tels qu'hydroxybenzotriazole ou imidazole (F.H.C. STEWART, Aust. J. Chem. 21 (1968) 1639; M. CHOREV, Y. KNOBLER, Y.S. KLAUSNER, J. chem. Research. (1977) 2246), celle au dicycholexylcarbodiimide en présence de catalyseurs, tels que diméthylaminopyridine (C. GILON, A. HASSNER, Y. CLAUSNER, Tetrahedron Lett. 40 (1979) 3811).

Le groupement de protection temporaire est alors éliminé, suivant une méthodologie adéquate, par exemple pour le groupement BOC par action d'une solution normale d'acide chlorhydrique dans l'acide acétique glacial. Le dérivé ainsi obtenu peut être couplé suivant les méthodes connues en synthèse peptidique avec un deuxième acylaminoacide pour donner un composé dipeptidique de formule $A_2$-$A_1$-Z-. Ainsi peut être réalisée la synthèse de la séquence peptidique des dérivés glycopeptidiques revendiqués, par addition séquentielle de chacun des résidus d'acides aminés, puis la synthèse des dérivés glycopeptidiques eux-mêmes, en utilisant par exemple l'acide α-0-benzyl-4,6-0-benzylidène-N-acétylmuramique. Les dérivés glycopeptidiques sont finalement obtenus à l'état libre, après élimination (par hydrogénolyse par exemple) des groupements protecteurs.

Une autre voie, préférée, consiste à coupler directement le dérivé α-substitué (par un amide ou un ester, suivant la formule générale) de l'acide N-acétyl-muramyl-L-alanyl-D-glutamique avec le reste N-$(A)_n$-Z, préalablement préparé, par exemple, mais de façon non limitative, en utilisant la méthode aux anhydrides mixtes.

b) Séquence de synthèse des composés glycopeptidiques correspondant à la variante de la formule générale dans laquelle n est égal à zero

Lorsque n est égal à zéro, la fonction γ-carboxyle du résidu D-glutamyle est engagée dans une liaison ester avec le reste Z. Les méthodes décrites ci-dessus (paragraphe a) sont alors employées pour préparer un dérivé du type acyl-D-glutamique α-(amide ou ester) γ-Z, ou bien directement un dérivé dipeptidique du type acyl-X-D-glutamique α-(amide ou ester)γ-Z. Ces dérivés après élimination du groupement acyle, sont couplés avec un dérivé adéquat de l'acide muramique, tel que l'acide α-0-benzyl-4-6-0-benzylidène-N-acétvl-muramique. Les dérivés glycopeptidiques selon l'invention sont finalement obtenus après, par exemple, hydrogénolyse des groupements de protection temporaire.

D'autres caractéristiques de l'invention apparaîtront encore au cours de la description qui suit, de l'un de ses exemples préférés, en l'absence de toute intention limitative.

A) Préparation du N-acétyl-muramyl-L-alanyl-D-isoglutaminyl-L-alanyl -cholestéryl ester (III)

a) N-benzyloxycarbonyl-L-alanyl-cholestéryl ester (I)

1,546 g (4 mmoles) de cholestérol, 1,12g (5 mmoles) de Z-L-alanine, 500 mg (4 mmoles) de diméthylaminopyridine, sont dissous dans 20 ml de diméthylformamide et 5 ml de tétrahydrofurane. A 0° C, on ajoute 1,112 g (5,4 mmoles) de dicyclohéxylcarbodiimide. Le mélange réactionnel est agité pendant 1 nuit, à température ordinaire, puis la dicyclohéxylurée précipitée est filtrée, et le filtrat concentré à sec. Le résidu obtenu est repris dans le chlorure de méthylène, puis lavé avec $NaHCO_3$M, $H_2O$, $KHSO_4$ 5 %, $H_2O$, et séché sur $MgSO_4$. Après concentration à sec, le produit est purifié sur colonne de silice, l'élution étant réalisée avec le système de solvants toluène/éther (10/1, v/v). Les fractions sont testées par chromatographie sur couche mince de gel de silice dans le système de solvants benzène/éther (7/3, v/v), celles contenant le produit étant rassemblées, concentrées et lyophilisées à partir de leur solution dans le dioxane: 1,63 g (70 %).

$[\alpha]^{30°}_D$ = - 32° (c = 1, chloroforme)

Analyse élémentaire: calculée pour $C^{38}H_{57}NO_4$, 0,25 dioxane: C % 76,3 - H % 9.7 - N % 2,3: Trouvé C % 76,3 - H % 9,5 - N % 2,3.

b) [-alanyl-cholesteryl ester, acétate (II)

284 mg (0,48 mmoles) de N-benzyloxycarbonyl-L-alanyl-cholestérylester dissous dans 20 ml de tétrahydrofurane sec et depéroxydé, et 3 ml d'acide acétique glacial. L-hydrogénation est poursuivie pendant 2 heures en présence de 300 mg de Pd 5 % sur charbon. Le catalyseur est filtré et le produit après concentration est lyophilisé à partir de sa solution dans le dioxane: 226 mg (91 %).

$[\alpha]^{25}_D$ = - 18,75° (c = 0,4, chloroforme).

Analyse élémentaire: calculé pour $C_{30}H^{51}NO_2$, $CH_3COOH$, $0,25H_2O$: C % 73.6 - H % 10.7 - N % 2,7: Trouvé C % 73,5 - H % 10,7 - H % 2.5.

c) N-acetyl-muramyl-L-alanyl-D-isoglutaminyl-L-alanyl-cholestéryl ester (III) (ou MTP-chol)

1) 98,5 mg (0,2 mmoles) de N-acétyl-muramyl-L-alanyl-D-isoglutamine sont incubés à température ambiante, pendant 1 heure, dans 5 ml de diméthyl formamide, avec 85 mg (0,2 mmoles) de N-cyclohexyl-H-(β-(N-méthylmorpholino)éthyl]-carbodiimide, p-toluène sulfonate et 32 mg (0,2 mmoles) d'hydroxybenzotriazole. Puis 103,56 mg (0,2 mmoles) de L-alanyl-cholestéryl ester, acétate et 22 µl (0,2 mmoles) de N-méthyl-morpholine, dans 3 ml de chlorure de méthylène sont ajoutés. Après 48 heures, le mélange réactionnel est concentré à sec et le produit purifié sur colonne de silice, l'élution étant réalisée avec le mélange de solvants chloroforme /méthanol (4/1, v/v): 45 mg (25 %).

2) 221,6 mg (0,45 mmoles) de N-acétyl-muramyl-L-alanyl-D-isoglutamine sont repris puis concentrés à sec, deux fois, avec 20 ml de diméthylformamide sec. 2,5 ml de ce solvant sont finalement ajoutés: à la solution refroidie à -15°C, on ajoute 50 µl (0,45 mmoles) de N-méthylmorpholine et 60 µl (0,45 mmoles) de chloroformiate d'isobutyle. Après 3 minutes, on ajoute en solution dans 3 ml de tétrahydrofuranne 210 mg (0,4 mmoles) de L-alanyl-cholestéryl ester, acétate et 45 µl (0,4 mmoles) de N-méthylmorholine. Le mélange réactionnel est laissé à -15° pendant 1 nuit, puis est concentré à sec et purifié comme décrit ci-dessus. Le produit est obtenu après lyophilisation à partir de sa solution dans l'acide acétique glacial: 215 mg (57,7 %).

La pureté du produit est vérifiée par chromatographie en couche mince sur silice dans le système de solvants chloroforme/méthanol (4/1, v/v) et chloroforme/tétrahydrofurane /méthanol (5/2/1, v/v); et par dosage des acides aminés et du cholestérol.

$[\alpha]^{25}_D$ = +3,9° (c = 0,5 acide acétique glacial).

Analyse élémentaire:

calculé pour $C_{49}H_{81}N_5O_{12}$,$2CH_3COOH$,$1H_2O$:

C % 59,5 - H % 8,6 - N % 6,6; Trouvé C % 59,7 - H % 8,5 - N % 6,9.

B) Propriétés biologiques du "MTP-chol"

1) Préparation des liposomes

Le PC (distearoyl PC de CALIBIO-CHEM) et le PS (PS de cerveau de boeuf de SIGMA) sont dissous dans du chloroforme et additionnés de MTP-chol dissous dans de l'éther/éthanol (4/1) à raison de 5 µg de MTP-chol par mg de PC + PS. La solution de lipides ainsi obtenue est placée dans un ballon et les solvants sont évaporés à l'évaporateur rotatif de façon à obtenir un film sur la surface du ballon.

Le film est hydraté et agité dans un dispositif de type VORTEX en présence d'une solution isotonique de NaCl tamponnée au phosphate (PBS "Phosphate Buffered saline"); additionné d'antibiotiques. Les liposomes ainsi obtenus sont centrifugés 30 minutes à 16 300 g puis remis en suspension dans du PBS + antibiotiques de façon à obtenir une concentration de lipides de 5 mg/ml.

Des liposomes vides, c'est-à-dire sans MTP-chol, sont préparés dans les mêmes conditions et servent de témoins.

2) Activation des macrophages par le MTP-chol dans des liposomes

Les macrophages utilisés sont des macrophages péritonéaux de souris BDF 1 femelles de huit semaines. Ils ont été appelés dans la cavité péritonéale par l'injection i.p. 4 jours plus tôt, de 1,5 ml d'un irritant inflammatoire stérile, le milieu au thioglycollate (commercialisé par Institut Pasteur Production).

Les cellules péritonéales sont obtenues par lavage péritonéal avec 5 ml de milieu MEM (voir plus bas) des souris préalablement tuées par dislocation cervicale et exsanguinées par décapitation.

Après centrifugation, elles sont remises en suspension dans du MEM (Minimal Essential Medium, Institut Pasteur Production) additionné de 5 % de sérum de veau foetal (gibco) inactivé, et d'antibiotiques (pénicilline + streptomycine).

Les macrophages sont identifiés au microscope par leur aptitude à endocyter le rouge neutre et comptés sur un aliquot.

La suspension est ensuite ajustée à $2,10^6$ macrophages/ml et répartie à raison de 250 µl par cupule dans une boîte de plastique Microtest II (Nunclon) de 96 cupules.

La boîte est placée dans une étuve (HERAEUS) à 37° en atmosphère air + 5 % $CO_2$ saturée d'eau.

Après une incubation pendant 4 heures, les macrophages ont adhéré; les cellules non adhérentes (lymphocytes essentiellement) sont éliminées par aspiration du milieu d'incubation (à la seringue) et deux lavanes au PBS. Les cupules sont ensuite additionnées de 250 µl de milieu contenant ou non des liposomes (vides ou contenant du MTP-chol).

Après 24 heures d'incubation les cupules sont lavées par du PBS puis remplies par 250 µl d'une suspension de cellules du mastocytome $P_{815}$ (0,3 x $10^6$ cellules/ml), additionnées de thymidine tritiée (Saclay) à une concentration de 1,2 µM (activité spécifique 1 Ci/mmole).

Le mastocytome $P_{815}$ est entretenu en ascite par repiquage, tous les 11 jours, dans la cavité péritonéale de souris $DBA_2$.

Après 24 heures de coculture, les cellules de chaque cupule sont récoltées sur filtre de fibres de verre grâce à un "récolteur" (Skatron, Lierbyen, Norvège): au cours de cette récolte, faite par lavage des cupules, par un courant d'eau distillée, l'ADN des cellules est libéré par lyse des cellules et se fixe sur le filtre. Si les cellules de $P_{815}$ se sont multipliées, elles ont incorporé de la thymidine tritiée et leur ADN est radioactif, la radioactivité étant proportionnelle à la croissance de la tumeur (les macrophages ne se multiplient pas dans ces conditions) (12).

La partie de filtre correspondant à chaque cupule est découpée et comptée en présence de liquide scintillant (lipolumac, Luma) dans un compteur à scintillation (Rackbeta, LKB). Chaque essai est fait en triple, le résultat considéré étant la moyenne de 3 cupules.

L'activité antitumorale des macrophages est exprimée en pourcentage d'inhibition de croissance: (% IC)

$$(\%) = \frac{T-X}{T} \times 100$$

T = incorporation de thymidine dans la coculture macrophanes non traités + cellules tumorales
X = incorporation de thymidine dans la coculture macrophages traités + cellules tumorales.

Le tableau ci-après donne le % d'inhibition de croissance de Mastocytome $P_{815}$ cocultive en présence de macrophages traités par des liposomes vides ou des liposomes contenant 5 µn de MTP-chol/mg de lipides.

**Tableau**

| | | 400 µg lipides/ml % Pc | 200 µg lipides/ml % Pc |
|---|---|---|---|
| Liposomes vides | 1er essai | 0 | 10,7 |
| | 2ème essai | 1 | 11,5 |
| Liposomes + 5 µg MTP-chol/mg de lipide | 1er essai | 60 | 64,6 |
| | 2éme essai | 63 | 71,5 |
| MDP en solution 50 µg/ml | | 18 | |

La présence de MTP-chol dans des liposomes, à une concentration de 2 µg/ml (400 µg liposomes/ml de milieu) ou 1 µg/ml (200 µg liposomes/ml de milieu) active les macrophages significativement plus que ne le font 50 µg de MDP.

3) <u>Activation des macrophages par le MTP-chol dans les liposomes-activité cytotoxique</u>.

Les macrophages utilisés sont les macrophages alvéolaires de rats F344 mâles de 200 gm. Les macrophages sont obtenues par un lavage du poumon avec 9 x 5 ml de milieu PBS de Dulbecco, sans calcium ou magnésium, de rats anesthésiés avec 0,5 ml 5 % Nembutal ip (Laboratoires Abbott S.A.) et exsanguinés par incision d'une artère du rein. Après centrifugation, elles sont remises en suspension dans du MEM (Minimal Essential Medium) additionné de 5 % de sérum de veau foétal inactivé (Gibco), glutamine, pyruvate de sodium, acides aminés non-essentiels, vitaminés et d'antibiotiques (penicilline et streptomycine).

La suspension est ensuite ajustée à 5 x $10^5$ macrophages/ml et répartie à raison de 100 µl par cupule dans une boîte de plastique Titertek de 96 cupules. Après une incubation pendant 4 heures, les macrophages ont adhéré; les cellules non-adhérentes (moins de 5 %) sont éliminées par aspiration du milieu et 2 lavages avec le milieu d'incubation. Les cupules sont ensuite additionnées de 200 µl de milieu contenant ou non des liposomes (vide ou contenant du MTP-chol) ou MDP en solution.

Après 24 heures d'incubation des macrophages, les cupules sont lavées 3 fois, et puis remplies par 100 µl milieu. Les cupules sont ensuite additionnées de 100 µl d'une suspension de cellules du mélanome B16-BL6 (5 x $10^4$ cellules/ml) marquées par $^{125}$I-deoxyuridine.

Le mélanome B16-BL6, une sous-lignée du mélanome B16, est entretenu en culture in vitro.

Après 96 heures de coculture, les cellules mortes sont éliminées par 3 lavages de chaque cupule avec 200 µl

de PBS de Dulbecco. Les cupules sont ensuite additionnées de 200 µl de 0,5 M NaOH. Le DNA des cellules est libéré par lyse des cellules. Si les cellules sont tuées par les macrophages, leur DNA est libéré dans le milieu, et éliminé par les levages avant l'addition de NaOH (Effects of liposome structure and liposome composition on the activation of the tumoricidal properties of macrophages by liposomes containing muramyl dipeptide. Schroit, A.J. and Fidler, I.J. Cancer Research, 42, 161 - 167 (1982)). La radioactivité restant dans chaque cupule est comptée dans un compteur gamma (Beckman Gamma 4000). Chaque essai est fait en triple, le résultat considéré étant la moyenne des 3 cupules.

L'activité antitumorale des macrophages est exprimée en pourcentage de cytotoxicité (% CYT):

$$\% \text{ CYT} = \frac{T-X}{T} \times 100$$

ou T = $^{125}$I-deoxyuridine restant dans la coculture macrophages non-traités + cellules tumorales;
et X = $^{125}$I-deoxyuridine restant dans la coculture macrophages traités + cellules tumorales.

**Tableau**

Le tableau donne le % CYT du mélanome B16-BL6 cocultivé en présence de macrophages traités par les liposomes vides ou des liposomes contenant 0,04, 0,4 et 4,0 µg MTP-mg phospholipide.

La présence de MTP-chol dans les liposomes, à une concentration de plus de 0,017 µg/ml (400 µg phospholipide/ml) ou de 0,0017 µg/ml (40 µg phospholipide/ml) active significativement les macrophages. Le même degré d'activation est obtenu pour le MDP en solution à une concentration de 10 à 500 µg/ml.

| Traitement | | MTP-chol µ/ml | % CYT, 400 µg phospholi-pide/ml | MTP-chol µ/ml | % CYT, 40 µg phospholi-pide/ml |
|---|---|---|---|---|---|
| Liposomes vides | 1° essai | - | 6 | - | 0 |
| | 2° essai | - | 9 | - | 0 |
| Liposomes + 4 µg MTP-chol/mg de phospholipide | 1° essai | 1,7 | 30 | 0,17 | 54 |
| | 2° essai | | 38 | | 52 |
| Liposomes + 0,4 µg MTP-chol/mg de phospholipide | 1° essai | 0,17 | 23 | 0,017 | 21 |
| | 2° essai | | 29 | | 18 |
| Liposomes + 0,04 µg MTP-chol/mg de phospholipide | 1° essai | 0,017 | 9 | 0,0017 | 17 |
| | 2° essai | | 16 | | 14 |

| Concentration de MDP en solution | Degré d'activation des macrophages |
|---|---|
| 500 µg/ml | 52 |
| 100 µg/ml | 32 |
| 10 µg/ml | 18 |
| 1 µg/ml | 6 |
| 0,1 µg/ml | 0 |

Les résultats obtenus avec l'un des représentants de la classe de composés selon l'invention montrent que la conjugaison d'un stérol à un muramyl peptide rend ce dernier particulièrement apte à activer les macrophages jusqu'à leur conférer une forte activité anti-tumorale, après une inclusion facile de ce conjugué dans des liposomes.

Il est à remarquer également que les conjugués stérols-muramylpeptides ont également conservé des propriétés adjuvantes et, en même temps, anti-infectieuses (notamment à l'égard des Klebsiella) caractéristiques du MDP.

Les composés selon l'invention ont également des propriétés antivirales, comme en témoignent les résultats d'essais qui suivent, en rapport avec l'un des composés représentatifs de la classe selon l'invention, à savoir le MTP-cholestérol.

L'activité antivirale a été évaluée dans un système d'infection expérimentale. On utilise des souris Swiss âgées de 10 semaines. Le virus choisi est celui du virus influenza A/PR$^{8}$.

Au jour J-0, les animaux reçoivent par voie intranasale 50 microlitres d'une dilution virale au 1/10$^{4}$.

Au jour J-1, action préventive, ou J+1, action curative, ils reçoivent le produit à tester, en l'occurence le MDP-L-Ala-cholestérol (IC 83 1347).

Plusieurs voies d'administration du médicament ont été utilisées: voie orale, voie intranasale, voie souscutanée.

Les doses administrées ont été les suivantes:

. par voie orale ou par voie intranasale, 1 mg par souris,
. par voie sous-cutanée, 0,5 mg par souris.

Une activité antivirale du MTP-cholesterol a été mise en évidence tant à titre préventif qu'à titre curatif. C'est ainsi qu'à titre préventif, l'administration de 1 mg. du produit au J-1 par voie intranasale entraîne notamment une survie de 67 % des animaux.

A titre curatif, les meilleurs résultats sont obtenus par une administration de 0,5 mg du produit au J+1 par voie sous-cutanée qui entraîne le survie de 71 % des souris.

Ces résultats soulignent donc, outre l'existence d'une activité antivirale curative du MTP-cholestérol, l'intérêt des voies locales d'administration de ce produit.

L'invention concerne donc des réactifs biologiques qui peuvent être constitués à l'aide des composés selon l'invention. Ces réactifs sont utiles comme composés de référence ou de comparaison pour l'étude des propriétés d'activation de macrophages de composés à l'étude.

L'invention est également relative à des médicaments renfermant à titre de principe actif au moins l'un des composés selon l'invention, ces médicaments étant applicables en tant qu'agents de stimulation des défenses immunitaires, plus particulièrement d'agents stimulant la résistance antitumorale et/ou antivirale du sujet auquel ils sont administrés.

Les médicaments selon l'invention peuvent être administrés à un hôte - animal ou être humain - de toute façon appropriée à l'obtention de l'effet désiré.

L'invention concerne naturellement aussi les diverses compositions pharmaceutiques, plus particulièrement à base de liposomes mettant en jeu des lipides physiologiquement acceptables, auxquelles les composés selon l'invention peuvent être incorporés, le cas échéant en association avec d'autres substances activés.

Des compositions pharmaceutiques avantageuses sont constituées par des suspensions de liposomes injectables contenant une dose efficace d'au moins un composé selon l'invention. De préférence, ces suspensions sont réalisées dans une phase aqueuse stérilisée isotonique, de préférence saline ou glucosée.

L'invention concerne plus particulièrememt de telles suspensions qui sont aptes à être administrées par injection intradermiques, intramusculaires ou sous-cutanées, intraveineuse ou encore par scarifications.

L'invention concerne aussi des compositions pharmaceutiques, de préférence sous forme de liposomes, administrables par d'autres voies, notamment par voie orale ou rectale, ou encore sous des formes destinées à venir en contact avec des muqueuses, notamment les muqueuses oculaires, nasales, pulmonaires ou vaginales.

En conséquence, elle concerne des compositions pharmaceutiques dans lesquelles l'un au moins des composés selon l'invention se trouve associé à des excipients pharmaceutiquement acceptables, solides ou liquides, adaptés à la constitution de formes d'administration orale, oculaire ou nasale ou avec des excipients adaptés à la constitution de formes d'administration rectale, ou encore avec des excipients adaptés à l'administration vaginale, par exemple gélatineux. Elle concerne enfin des compositions destinées à la voie pulmonaire, notamment des solutions préparées en vue de l'administration au moyen d'un, appareil d'aérosol classique.

A titre d'exemples de doses susceptibles d'être administrées, pour renforcer les défenses anti-tumorales et/ou antivirales de l'hôte, on mentionnera des doses de 0,1 à 1000 µg par kg de corps, par exemple de 0,1 à 100 µg lorsque l'administration est effectuée par voie parentérale, ou encore d'une dose de 1 à 1000 µg par kg de corps, par voie orale. Ces doses sont exprimées en dérivé du MDP inclus dans le liposome.

Ces compositions peuvent être utilisées pour réaliser des injections intra-lésionnelles dans des tumeurs du type tumeurs mammaires, mélanomes et autres tumeurs solides.

L'invention ne se limite évidemment pas aux modes de réalisation décrits ci-dessus à titre d'exemple et l'homme de l'art peut y apporter des modifications sans pour autant sortir du cadre des revendications ci-après.

En particulier, elle concerne tous les conjugués de stérols-muramylpeptides qui ne se distinguent de ceux dont les formules entrent plus particulièrement dans le cadre des revendications qui suivent que par des substitutions qui n'ont qu'une importance secondaire. Il en est ainsi par exemple des composés dans lesquels le noyau saccharidique de la partie muramyl-peptide est substitué, par exemple en position 6. Des exemples de telles substitutions ont été indiqués notamment dans les brevets antérieurs cités plus haut (que ce soit en position 6 ou dans une autre position du cycle saccharidique, ou en une position de la chaîne peptidique). Il en est encore ainsi de ceux des conjugués de stérol-muramyl-peptides dans lesquels la liaison entre le groupe peptide et le groupe stérol, se fait par l'intermédiaire d'une fonction o1 - ou d'une fonction différente - située en une position autre que la position 3 sur le noyau poly-nucléaire, ou encore sur la chaîne carbonée éventuellement fixée sur le noyau à la position 17.

Ci-après la bibliographie à laquelle référence a été faite dans la présente demande.

Il va également de soi que les revendications qui suivent concernent aussi bien les composés qui y sont identifiés en tant que tels, que les sels qu'ils sont susceptibles de former, notamment des sels physiologiquement ou pharmaceutiquement acceptables.

**Bibliograpie**

1 - I.J. Fidler, Z. Barnes, W.E. Fogler, R. Kirsh, P. Bugelski and G. Poste,
Involvement of macrophages in the eradication of established metastases following intravenous injection of liposomes containing macrophage activators.

Cancer Res., 42, 496 - 501 (1982).

2 - D. Juy and L. Chedid,
Comparison between macrophage activation and enhancement of non-specific resistance to tumors by mycobacterial immunoadjuvants.
Proc. Nat. Acad. Sci., 72, 4105 - 4109 (1975).

3 - J.P. Tenu, E. Lederer and J.F. Petit,
Stimulation of thymocyte mitogenic protein secretion and of cytostatic activity of mouse peritoneal macrophages by trehalose dimycolate and muramyldipeptide.
Eur. J. Immunol., 10, 647 - 653 (1980).

4 - I.J. Fidler, S. Sone, W.E. Fogler and Z.L. Barnes,
Eradication of spontaneous metastases and activation of alveolar macrophages by intravenous injections of liposomes containing muramyl dipeptide.
Proc. Natl. Acad. Sci. USA, 78, 1680 - 1684 (1981).

5 - J.P. Tenu, A.C. Roche, A. Yapo, C. Kieda, M. Monsigny and J.F. Petit.
Absence of cell surface receptors for muramylpeptides in mouse peritoneal macrophages.
Biol. Cell. 44, 157 - 184 (1982).

6 - M. Parant, F. Parant, L. Chedid, A. Yapo, J.F. Petit and E. Lederer,
Fate of the synthetic immunoadjuvant, muramyl dipeptide ($^{14}C$-labelled) in the mouse.
Int. J. Immunopharmac., 1, 35 - 41 (1979).

7 - A. Yapo, J.F. Petit, E. Lederer, M. Parant, F. Parant and L. Chedid,
Fate of two $^{14}C$ labelled muramyl peptides: Ac-mur-L-Ala-γ-D-Glu-meso-$A_2$PM and Ac-Mur-L-Ala-γ-D-Glu-meso-$A_2$PM-D-Ala-D-Ala in mice. Evaluation of their ability to increase non specific resistance to Klebsiella infection.
Int. J. Immunopharmac. 4, 143 - 149 (1982).

8 - G. Poste, C. Bucana. A. Raz, P. Bugelski, R. Kirsh and I.J. Fidler,
Analysis of the fate of systematically administered liposomes and implication for their use in drug delivery.
Cancer Res., 42, 1412 - 1422 (1982).

9 - A.J. Schroit and I.J. Fidler,
Effects of liposome structure and lipid composition on the activation of the tumoricidal properties of macrophages by liposomes containing muramyl dipeptide.
Cancer Res., 42, 161 - 167 (1982).

10 - A.J. Schroit. E. Galligioni and I.J. Fidler,
Factors influencing the in situ activation of macrophages by liposomes containing muramyl dipeptide.
Biol. Cell, 47, 87 - 94 (1983).

11 - A.J. Schroit and I.J. Fidler,
Stimulation of macrophage-mediated destruction of tumor cells by liposomes containing a lipophilic derivative of muramyl dipeptide.
In E. Serou (ed.) Current concepts in human immunology and cancer immunomodulation, pp. 631 - 637. New York, Elsevier Biomedical Press, B.V., 1982.

12 - M. Lepoivre. J.P. Tenu, G. Lemaire and J.F. Petit,
Antitumor activity and hydrogen peroxide release by macrophage elicited by trehalose diesters.
J. of Immunol., 129, 860 - 866 (1982).

**Revendications** pour les états contractants: BE, CH, DE, FR, GB, IT, LI, LU, NL, SE

1. Composé résultant essentiellement d'une conjugaison covalente d'un muramylpeptide comportant une fonction amine et d'un stéroïde à tropisme membranaire comportant une fonction hydroxyle ou amine, tel que le cholestérol ou un précurseur de sa biosynthèse, ou encore un sitostérol, un stigmasterol, la prégnenolone, l'androstérone et l'oestrone.

2. Composé caractérisé par la formule générale suivante:

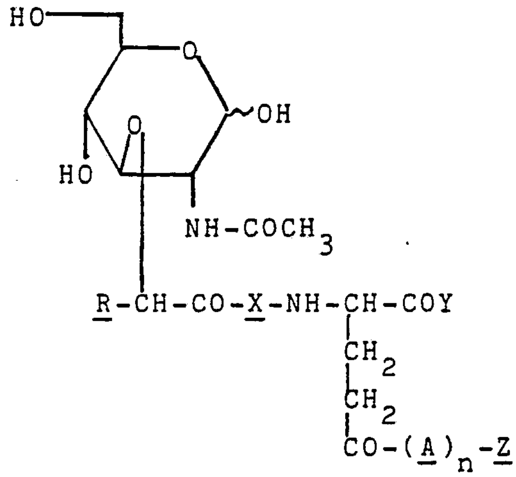

dans laquelle les substituants R, X, Y, A et Z ont l'une des significations suivantes:

- R est soit un atome d'hydrogène, soit un groupement alcoyle comprenant de 1 à 5 atomes de carbone,
- X est un résidu aminoacyle du groupe comprenant: L-alanyl, glycyl, L-valyl, L-isoleucyl, L-norleucyl, L-leucyl, L-seryl, L-threonyl, L-prolyl-, L-glutaminyl, L-asparaginyl, L-methionyl, L-tryptophanyl, L-phenylalanyl, L-tyrosyl;
- Y est un groupement $NH_2$ ou OH, ou encore un résidu alkyl comprenant de 1 à10 atomes de carbone;
- n = 0 ou un nombre entier de 1 à 5;
- A est (lorsque n prend les valeurs de 1 à 5) un résidu aminoacyle du groupe indiqué ci-dessus, mais aussi de formule telle que $-NH-(CH_2)_x-CO-$, avec des valeurs de x comprises entre 2 et 10, étant entendu que les groupes A présents dans un même composé peuvent être identiques ou différents;
- Z est un dérivé de 3-hydroxy androstane ou de 3-hydroxy androstène portant une fonction kétone ou une chaîne hydrocarbonée comprenant de 1 à 10, notamment de 2 à 8 atomes de carbone.

3. Composé selon la revendication 2, caractérisé en ce que la chaîne hydrocarbonée de Z est modifiée par une ou plusieurs fonctions cétone ol ou amine.

4. Composé selon la revendication 2, caractérisé en ce qu'il est constitué par un dérivé muramyl-dipeptideγ-L-alanyl-cholestéryl-ester.

5. Composé de formule selon la revendication 2:

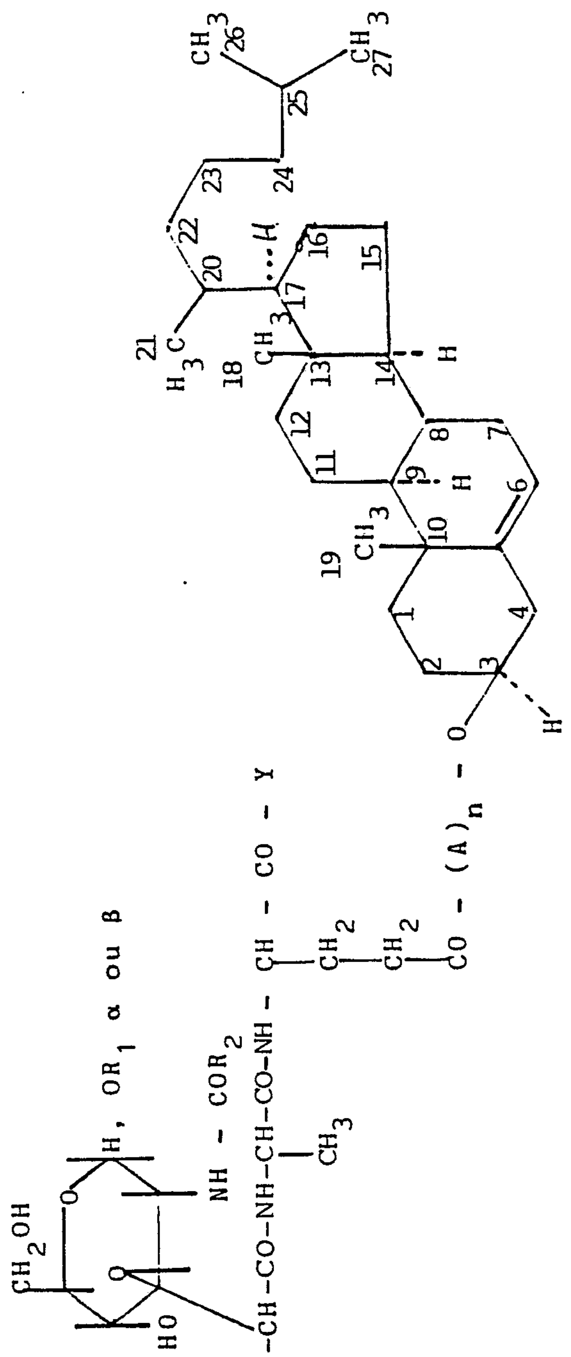

dans laquelle:

- $R_1$ représente l'hydrogène,
- $R_2$ représente le radical $-CH_3$,
- Y, n et A ont les significations indiquées à la revendication 2.

6. Liposomes contenant un composé selon l'une quelconque des revendications 1 à 5.

7. Liposomes selon la revendication 6, caractérisés en ce que leur teneur en lipides est à base de phospholipides.

8. Liposomes selon la revendication 7, caractérisés en ce que ces phospholipides sont à base de phosphatidylcholine et de phosphatidylsérine.

9. Composition ayant une action activatrice des macrophages in vivo leur conférant des propriétés de stimulation des défenses immunitaires, notamment des propriétés tumoricides et antivirales, cette composition consistant en une suspension de liposomes selon l'une quelconque des revendications 5 à 7, pharmaceutiquement acceptables en suspension dans un soluté aqueux.

10. Composition selon la revendication 9, caractérisée en ce qu'elle est injectable.

11. Utilisation des composés selon les revendications 1 à 5 pour la productiom de médicaments applicables en tant qu'agent de stimulation des défenses immunitaires, plus particulièrement d'agent stimulant de la résistance antitumorale ou antivirale ou les deux à la fois.

**Revendications** pour l'etat contractant: AT

1. Procédé de fabrication d'un composé résultant essentiellement d'une conjugaison covalente d'un muramylpeptide comportant un acide aminé à l'extrémité C-terminale de sa chaîne peptidique et d'un stéroïde à tropisme membranaire comportant une fonction hydroxyle, tel que le cholestérol ou un précurseur de sa biosynthèse ou encore un sitostérol, un stigmastérol, la prégnenolone, l'androstérone et l'oestrone, ce composé présentant la formule générale suivante:

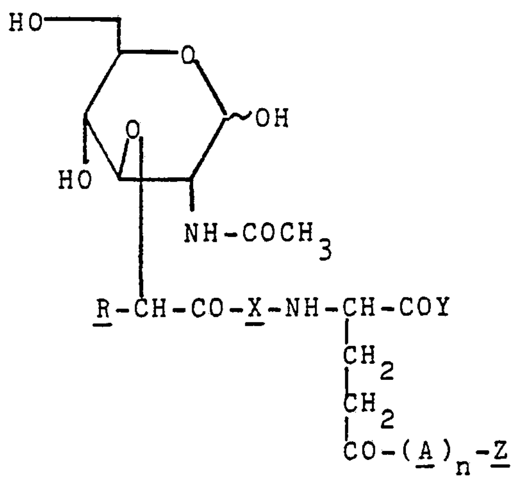

dans laquelle les substituants R, X, Y, A et Z ont l'une des significations suivantes:

- R est soit un atome d'hydrogène, soit un groupement alcoyle comprenant de 1 à 5 atomes de carbone,
- X est un résidu aminoacyle du groupe comprenant: L-alanyl, glycyl, L-valyl, L-isoleucyl, L-norleucyl, L-leucyl, L-seryl, L-threonyl, L-prolyl-, L-glutaminyl, L-asparaginyl, L-methionyl, L-tryptophanyl, L-phenylalanyl, L-tyrosyl;
- Y est un groupement $NH_2$ ou OH, ou encore un résidu alkyl comprenant de 1 à10 atomes de carbone;
- n = 0 ou un nombre entier de 1 à 5;
- A est (lorsque n prend les valeurs de 1 à 5) un résidu aminoacyle du groupe indiqué ci-dessus, mais aussi de formule telle que $-NH-(CH_2)_x-CO-$, avec des valeurs de x comprises entre 2 et 10, étant entendu que les groupes A présents dans un même composé peuvent être identiques ou différents;
- Z est un dérivé de 3-hydroxy androstane ou de 3-hydroxy androstène portant une fonction kétone ou une chaîne hydrocarbonée comprenant de 1 à 10, notamment de 2 à 8 atomes de carbone,

ce procédé étant caractérisé en ce que l'on fait réagir soit le stéroïde sus-indiqué de formule Z-OH avec muramylpeptide de formule

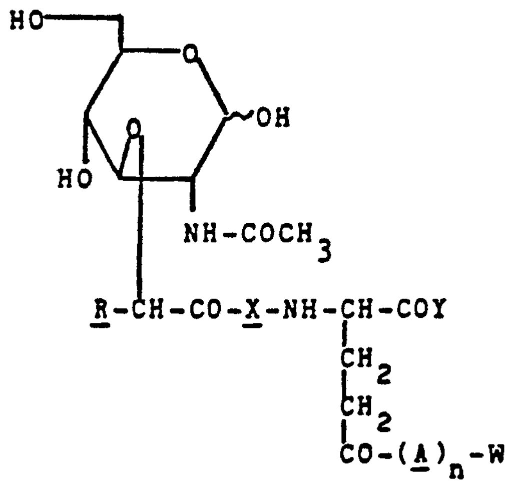

dans laquelle:

- les groupes R, X, Y, n, A ont les significations sus-indiquées et
- W représente un groupe de protection temporaire, tel que le groupe t-butyloxycarbonyle, utilisé en synthèse peptidique,
- soit un muramylpeptide ayant la susdite formule générale, dans laquelle:

- R, X, Y et W ont les significations susindiquées,
- n = 0,

avec un stéroïde modifié de formule H-$(A)_n$-Z, dans laquelle Z, A et n ont les significations sus-indiquées, la susdite réaction étant conduite dans des conditions propres à permettre la réalisation d'une liaison peptidique entre le muramylpeptide et le stéroïde.

2. Procédé selon la revendication 1, caractérisé en ce que Z est un dérivé de 3-hydroxy androstane ou de 3-hydroxy androstène portant une fonction kétone ou une chaîne hydrocarbonée comprenant de 1 à 10, notamment de 2 à 8 atomes de carbone.

3. Procédé selon la revendication 1 ou la revendication 2, caractérisé en ce que la chaîne hydrocarbonée de Z est modifiée par une ou plusieurs fonctions cétone, o1 ou amine, ces groupes ayant été protégés avant la mise en oeuvre de la susdite réaction.

4. Procédé selon la revendication 1, caractérisé en ce que le groupe X de muramylpeptide est un groupe alanyle et en ce que le stéroïde est constitué par le 1,4-hydroxy-androstane ou le 1,3-hydroxy-androstène.

5. Procédé selon l'une quelconque des revendications 1 à 4, caractérisé par l'étape suivante que constitue l'incorporation du composé obtenu à des liposomes.

6. Procédé selon la revendication 5, caractérisé en ce que leur teneur en lipides est à base de phospholipides.

7. Procédé selon la revendication 6, caractérisé en ce que ces phopholipides sont à base de phosphatidylcholine et de phosphatidylsérine.

**Patentansprüche** für die Vertagsstaaten: BE, CH, DE, FR, GB, IT, LI, LU, NL, SE

1. Verbindung, resultierend im wesentlichen aus einer kovalenten Bindung von einem Muramylpeptid, das eine Aminfunktion enthält, und von einem Steorid zum Membran-Tropismus, das eine Hydroxyl- oder Aminfunktion enthält, wie das Cholesterin oder ein Vorläufer seiner Biosynthese, oder ein Sitosterin, ein Stimasterin, das Pregnenolon, das Androsteron und das Östron.

2. Verbindungm gekennzeichnet durch die folgende allgemeine Formel

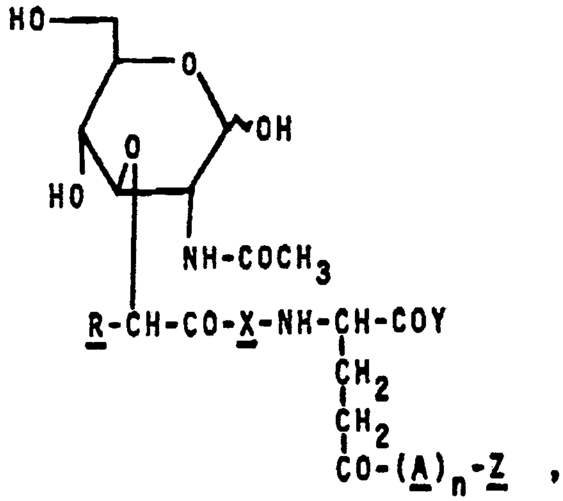

in welcher die Substituenten R, X, Y, A und Z eine der folgenden Bedeutungen haben:

- R ist entweder ein Wasserstoffatom oder eine Alkylgruppe, bestehend aus 1 bis 5 Kohlenstoffatomen;
- X ist ein Aminosäurerest der Gruppe bestehend aus: L-Alanyl, Glycyl, L-Valyl, L-Isoleucyl, L-Norleucyl, L-Leucyl, L-Seryl, L-Threonyl, L-Prolyl, L-Glutaminyl, L-Asparaginyl, L-Methionyl, L-Tryptophanyl, L-phenylalanyl, L-Tyrosyl;
- Y ist eine $NH_2$- oder OH-Gruppe oder auch ein Alkylrest, bestehend aus 1 bis 10 Kohlenstoffatomen;
- n = 0 oder eine ganze Zahl von 1 bis 5;
- A ist (wenn n die Werte von 1 bis 5 annimmt) ein Aminosäurerest der oben angeführten Gruppe, aber auch der Formel wie $-NH-(CH_2)_x-CO-$, mit den Werten von x zwischen 2 und 10 einschließlich, wobei als vereinbart gilt, daß die in derselben Verbindung anwesenden Gruppen A identisch oder verschieden sein können;
- Z ist ein Derivat von 3-Hydroxy-Androstan oder von 3-Hydroxy-Androsten, das eine Ketofunktion oder eine Kohlenwasserstoffkette, bestehend aus 1 bis 10, insbesondere aus 2 bis 8 Kohlenstoffatomen, trägt.

3. Verbindung nach Anspruch 2, dadurch gekennzeichnet, daß die Kohlenwasserstoffkette von Z durch eine oder mehrere Keto-, Ol- oder Aminofunktionen modifiziert ist.

4. Verbindung nach Anspruch 2, dadurch gekennzeichnet, daß sie durch ein Derivat Muramyl-dipeptid-γ-L-alanyl-cholesterylester gebildet ist.

5. Verbindung der Formel nach Anspruch 2:

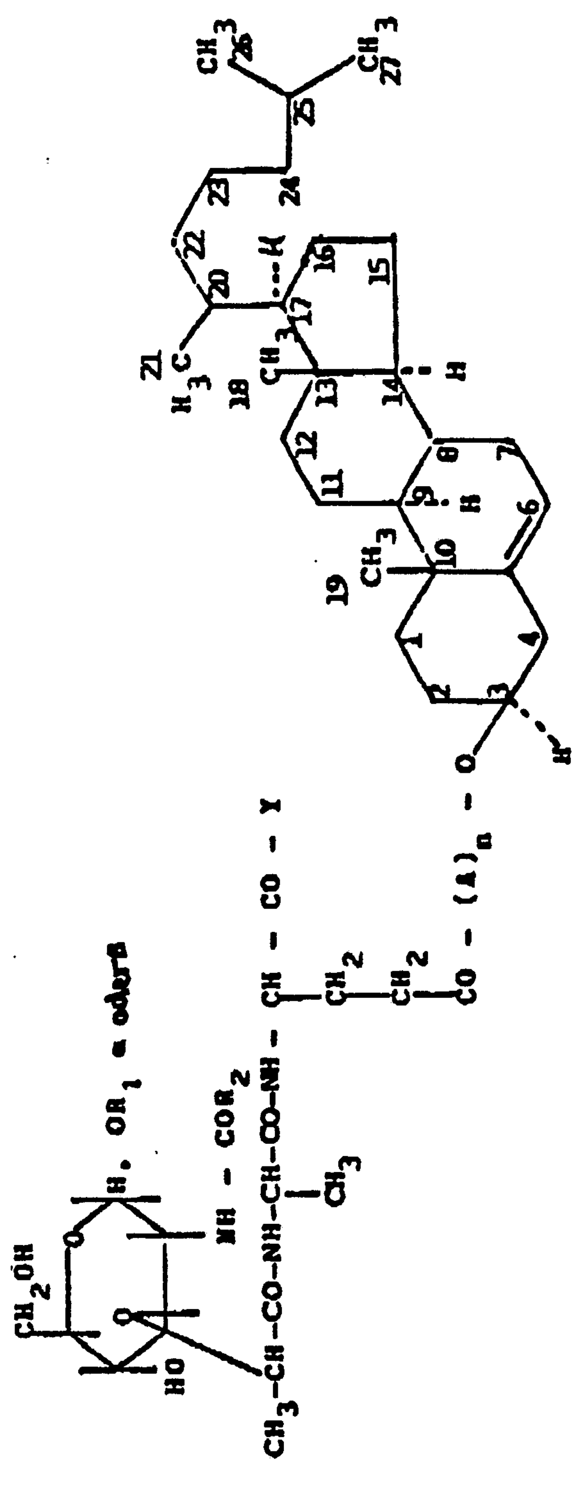

in der:

- $R_1$ Wasserstoff darstellt,
- $R_2$ das $CH_3$-Radikal darstellt,
- Y, n und A die in Anspruch 2 aufgeführten Bedeutungen haben.

6. Liposomen, enthaltend eine Verbindung nach einem der Ansprüche 1 bis 5.

7. Liposomen nach Anspruch 6, dadurch gekennzeichnet, daß ihr Gehalt an Lipiden auf Phospholipidbasis ist.

8. Liposomen nach Anspruch 7, dadurch gekennzeichnet, daß diese Phospholipide auf Phosphatidylcholin- und Phosphatidylserinbasis sind.

9. Zusammensetzung mit einer aktivierenden Wirkung auf Makrophagen in vivo, die ihnen Eigenschaften der Stimulation der Immunabwehr, insbesondere tumorizide und antivirale Eigenschaften verleiht, wobei diese

Zusammensetzung in einer Suspension von Liposomen nach einem der Ansprüche 5 bis 7 besteht, pharmazeutisch annehmbar in Suspension in einem wäßrigen Medium.

10. Zusammensetzung nach Anspruch 9, dadurch gekennzeichnet, daß sie injizierbar ist.

11. Verwendung der Verbindungen nach den Ansprüchen 1 bis 5 zur Herstellung von Medikamenten, die als Stimulationsmittel der Immunabwehr, insbesondere als Stimulationsmittel der antitumoralen oder antiviralen Resistenz oder von beiden zugleich anwendbar sind.

**Patentansprüche** für den Vertragsstaat: AT

1. Verfahren zur Herstellung einer Verbindung resultierend im wesentlichen aus einer kovalenten Bindung von einem Muramylpeptid, das eine Aminosäure am C-terminalen Ende einer Peptidkette enthält, und von einem Steroid zum Membran-Tropismus, das eine Hydroxylfunktion enthält, wie das Cholesterin oder ein Vorläufer seiner Biosynthese oder auch ein Sitosterin, ein Stigmasterin, das Pregnenolon, das Androsteron und das Östron, wobei diese Verbindung die folgende allgemeine Formel aufweist:

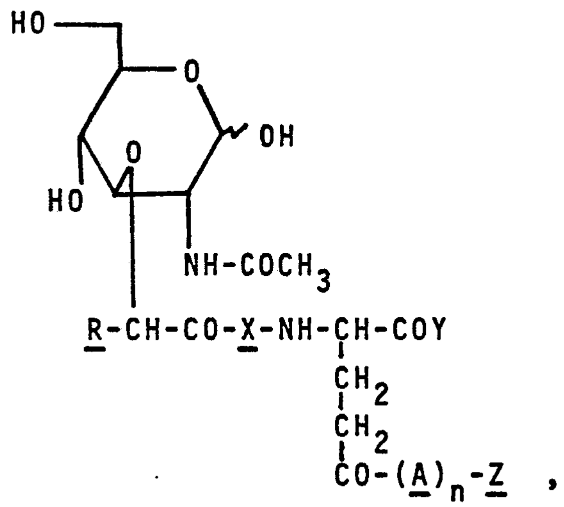

in welcher die Substituenten R, X, Y, A und Z eine der folgenden Bedeutungen haben:

- R ist entweder ein Wasserstoffatom oder eine Alkylgruppe, bestehend aus 1 bis 5 Kohlenstoffatomen;
- X ist ein Aminosäurerest der Gruppe bestehend aus: L-Alanyl, Glycyl, L-Valyl, L-Isoleucyl, L-Norleucyl, L-Leucyl, L-Seryl, L-Threonyl, L-Prolyl, L-Glutaminyl, L-Asparaginyl, L-Methionyl, L-Tryptophanyl, L-Phenylalanyl, L-Tyrosyl;
- Y ist eine $NH_2$- oder OH-Gruppe oder auch ein Alkylrest, bestehend aus 1 bis 10 Kohlenstoffatomen;
- n = 0 oder eine ganze Zahl von 1 bis 5;
- A ist (wenn n die Werte von 1 bis 5 annimmt) ein Aminosäurerest der oben angeführten Gruppe, aber auch der Formel wie $-NH-(CH_2)_x-CO-$, mit den Werten von x zwischen 2 und 10 einschließlich, wobei als vereinbart gilt, daß die in derselben Verbindung anwesenden Gruppen A identisch oder verschieden sein können,
- Z ist ein Derivat von 3-Hydroxy-AndrostaN oder von 3-Hydroxy-Androsten, das eine Ketofunktion oder eine Kohlenwasserstoffkette, bestehend aus 1 bis 10, insbesondere aus 2 bis 8 Kohlenstoffatomen, trägt.

dadurch gekennzeichnet, daß man reagieren läßt:

- entweder ein Muramylpeptid der Formel

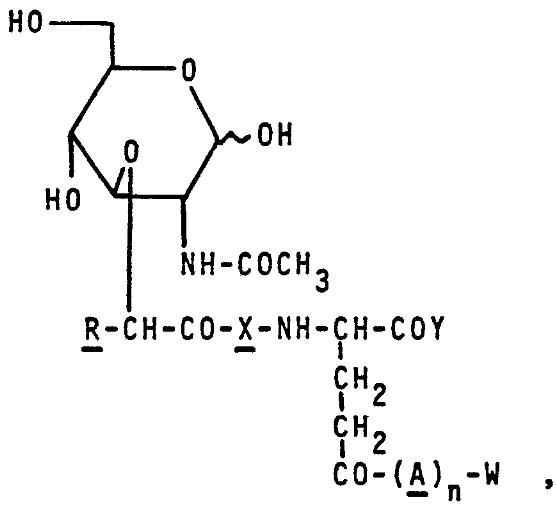

in welcher:

- die Gruppen R, X, Y, n, A die oben angeführten Bedeutungen haben und
- W eine temporäre Schutzgruppe wie die Gruppe t-Butyloxycarbonyl darstellt, verwendbar in der Peptidsynthese,

mit dem obengenannten Steorid der Formel Z-OH - oder ein Muramylpeptid mit der obengenannten allgemeinen Formel, in welcher:

- R, X, Y und W die oben angeführten Bedeutungen haben,
- n = 0,

mit einem modifizierten Steroid der Formel H-$(A)_n$-Z, in welcher Z, A und n die oben angeführten Bedeutungen haben,

wobei die obengenannte Reaktion unter Bedingungen durchgeführt wird, die die Realisierung einer Peptidbindung zwischen dem Muramylpeptid und dem Steroid erlauben.

2. Verfahren nach Anspruch 1, dadurch gekennzeichnet, daß Z ein Derivat von 3-Hydroxy-Androstan oder von 3-Hydroxy-Androsten ist, das eine Kohlenwasserstoffkette, bestehend aus 1 bis 10, insbesondere aus 2 bis 8 Kohlenstoffatomen, trägt.

3. Verfahren nach Anspruch 1 oder Anspruch 2, dadurch gekennzeichnet, daß die Kohlenwasserstoffkette von Z durch eine oder mehrere Keto-, Ol- oder Aminfunktionen modifiziert ist, wobei diese Gruppen vor dem Einsatz in der Durchführung der obengenannten Reaktion geschützt wurden.

4. Verfahren nach Anspruch 1, dadurch gekennzeichnet, daß die Gruppe X vom Muramylpeptid eine Alanylgruppe ist und daß das Steroid durch das 1,3-Hydroxy-Androstan oder das 1,3-Hydroxy-Androsten gebildet ist.

5. Verfahren nach einem der Ansprüche 1 bis 4, gekennzeichnet durch den folgenden Schritt, der die Einbringung der erhaltenen Verbindung in Liposomen darstellt.

6. Verfahren nach anspruch 5, gekennzeichnet durch einen Gehalt der Liposomen an Lipiden auf Phospholipidbasis.

7. Verfahren nach Anspruch 6, dadurch gekennzeichnet, daß diese Phospolipide auf Phosphatidylcholin- und Phosphatidylserinbasis sind.

**Claims** for the Contracting States: BE, CH, DE, FR, GB, IT, LI, LU, NL, SE

1. Compound resulting essentially from a covalent conjugation of a muramylpeptide having an amine group and of a steroid having membranal tropism and including a hydroxyl or amine group, such as cholesterol, or a precursor of its biosynthesis or further a sitosterol, a stigmasterol, pregnenolone, androsterone and oestrone.

2. Compoud characterised by the following general formula:

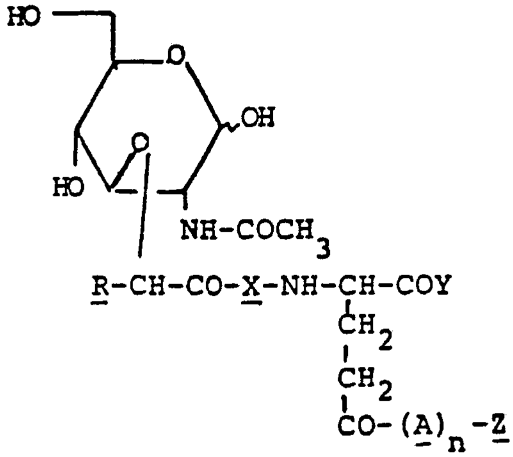

in which the substituents R, X, Y, A and Z have one of the following meanings:

- R is either a hydrogen atom, or an alkyl group comprising 1 to 5 carbon atoms,
- X is an aminoacyl residue of the group comprising: L-alanyl, glycyl, L-valyl, L-isoleucyl, L-norleucyl, L-leucyl, L-seryl, L-threonyl, L-prolyl, L-glutamyl, L-asparaginyl, L-methionyl, L-tryptophanyl, L-phenylalanyl, L-tyrosyl;
- Y is a $NH_2$ or OH group, or an alkyl residue comprising 1 to 10 carbon atoms;
- n = 0 or a whole number from 1 to 5;
- A is (when n takes the values 1 to 5) either an aminoacyl residue of the above-indicated group, or a residue of formula $-NH-(CH_2)_x-CO-$, with values of x comprised between 2 and 10, it being understood that the A groups present in the same compound can be identical or different;
- Z is a derivative of 3-hydroxy androstane or of 3-hydroxy-androstene bearing a ketone function or a hydrocarbon chain comprising 1 to 10, particularly 2 to 8 carbon atoms.

3. Compound according to claim 2, characterized in that the hydrocarbon chain of Z is modified by one or more ketone, ol or amine groups.

4. Compound according to claim 2, characterised in that it consists of a muramyl-dipeptide-γ-L-alanyl-cholesteryl-ester derivative.

5. Compound according to claim 2:

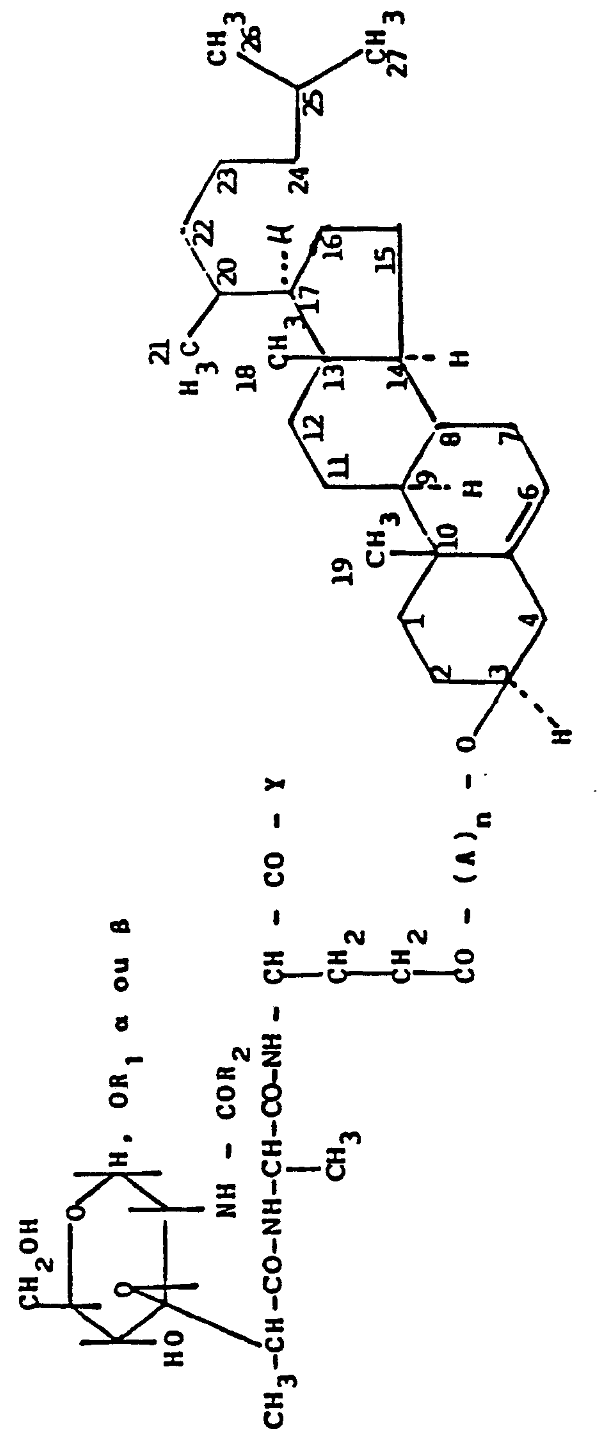

in which

- $R_1$ represents hydrogen
- $R_2$ represents the $-CH_3$ radical
- Y, n and A have the meanings indicated in claim 2.

6. Liposomes containing a compound according to any one of claims 1 to 5.

7. Liposomes according to claim 6 characterised in that their lipid content is based on phospholipids.

8. Liposomes according to claim 7, characterized in that the phospholipids are based on phosphatidylcholine and phosphatidylserine.

9. Composition having a macrophage activating action in vivo, conferring on them immunitary defense stimulation properties, more particularly tumoricidal and antiviral properties, said composition consisting of a

suspension of liposomes according to any one claims 5 to 7, pharmaceutically acceptable in suspension in an aqueous medium.

10. Composition according to claim 9, characterised in that it is injectable.

11. Use of compounds according to claims 1 to 5 for the production of medicaments applicable as immunitary defense stimulation agents, more particularly as antitumoral or antiviral resistance stimulation agents or both.

**Claims** for the Contracting State: AT

1. Process for producing a compound resulting essentially from a covalent conjugation of a muramylpeptide having an amino acid at the C-terminal extremity of its peptide chain and of a steroid having membranal tropism and including a hydroxyl group, such as cholesterol or a precursor of its biosynthesis or further a sitosterol, a stigmasterol, pregnenolone, androsterone and oestrone, this compound having the following general formula:

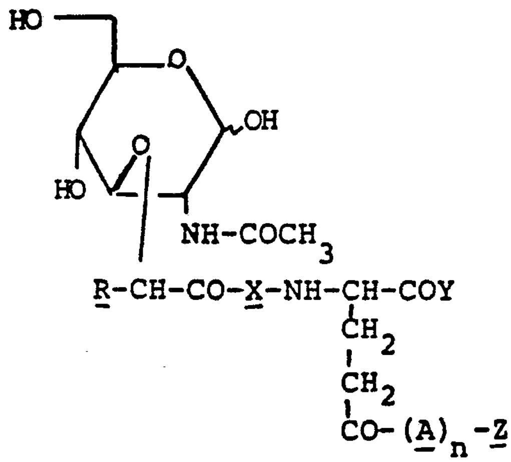

in which the substituents R, X, Y, A and Z have one of the following meanings:

- R is either a hydrogen atom, or an alkyl group comprising 1 to 5 carbon atoms,
- X is an aminoacyl residue of the group comprising: L-alanyl, glycyl, L-valyl, L-isoleucyl, L-norleucyl, L-leucyl, L-seryl, L-threonyl, L-prolyl, L-glutamyl, L-asparaginyl, L-methionyl, L-tryptophanyl, L-phenylalanyl, L-tyrosyl;
- Y is a $NH_2$ or OH group, or an alkyl residue comprising 1 to 10 carbon atoms;
- n = 0 or a whole number from 1 to 5;
- A is (when n takes the values 1 to 5) either an aminoacyl residue of the above-indicated group, or a residue of formula $-NH-(CH_2)_x-CO-$ with values of x comprised between 2 and 10, it being understood that the A groups present in the same compound can be identical or different;
- Z is a derivative of 3-hydroxy androstane or of 3-hydroxy-androstene bearing a ketone function or a hydrocarbon chain comprising 1 to 10, particularly 2 to 8 carbon atoms,

this process being characterised in that there is reacted together

either the above mentioned steroid having formula Z-OH with a muramyl peptide of the formula:

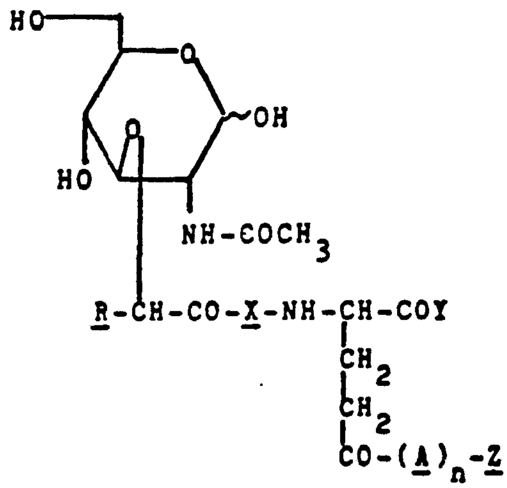

in which:

- the groups R, X, Y, n, A have the above-indicated meanings and
- W represents a temporary protection group such as the t-butyloxycarbonyl group used in peptide synthesis,

or a muramylpeptide having the above general formula in which:
- R, X, Y and W have the above-indicated meanings, n = 0,

with a modified steroid of the formula $H-(A)_n-Z$, in which Z, A and n have the above-indicated meanings, said reaction being carried out under conditions which allow the formation of a peptide bond between the muramylpeptide and the steroid.

2. Process according to claim 1, characterised in that Z is a derivative of 3-hydroxy androstane or of 3-hydroxy androstene and includes a ketone group or a hydrocarbon chain having 1 to 10, more particularly 2 to 8 carbon atoms.

3. Process according to claim 1 or claim 2, characterised in that the hydrocarbon chain Z is modified by one or more ketone, ol or amine groups, these groups having been protected before the initiation of the above reaction.

4. Process according to claim 1, characterised in that the X group of muramylpeptide is an alanyl group and in that the steroid consists of 1,3-hydroxy-androstane or 1,3-hydroxy-androstene.

5. Process according to any one of claims 1 to 9, characterised by the further step of incorporation of the obtained compound into liposomes.

6. Process according to claim 5, characterised in that their lipid content is of phospholipid base.

7. Process according to claim 6, characterised in that these phospholipids are of phosphatidylcholine or phosphatidylserine base.